# EUROPEAN PATENT APPLICATION

(11) **EP 4 266 054 A1**
(43) Date of publication of application: **25.10.2023**
(21) Application number: 21905969.8
(22) Date of filing: 17.12.2021
(51) Int. Cl.: G01N 33/50

(54) **IN VITRO METHOD FOR FUNCTIONAL ASSESSMENT OF HAEMATOPOIETIC PROGENITORS**

(30) Priority: 18.12.2020 CO 20015994
(71) Applicant: INSTITUTO DISTRITAL DE CIENCIA BIOTECNOLOGIA E INNOVACIÓN EN SALUD - IDCBIS, Bogotá (CO)
(72) Inventor: PERDOMO ARCINIEGAS, Ana Maria Del Pilar, Bogotá (CO); DEVIA MEJIA, Bellaneth, Bogotá (CO)
(74) Representative: Clarke, Modet y Cía., S.L.
(86) International application number: PCT/IB2021/061953
(87) International publication number: WO 2022/130332

(57) **Abstract**

The present invention relates to an in vitro method for functional assessment of haematopoietic progenitors which makes it possible to assess the ability of haematopoietic progenitor cells (HPCs) to respond to growth factors and proliferate in products used for HPC transplantation, without need for cell culture. The disclosed method can be used for functional quantification of haematopoietic progenitors pre-transplantation and can also be used as a functional assay or functional prognostic biomarker for leukaemia or other tumours. The method could be used to separate HPCs from cell mixtures and collect them for use in cell therapy.

## Description

### FIELD OF THE INVENTION

The present invention relates, in general, to cellular therapy, and in particular to the transplant of hematopoietic progenitor cells which are generally useful in the treatment of diseases which affect the hematopoietic physiology.

### PRIOR ART

The transplant of hematopoietic progenitor cells (HPC) from different sources is a cellular therapy based on the infusion of HPC as an implantation and replacement material of the hematopoietic material to treat any infirmity which affects the hematopoietic physiology.

After their infusion, it is necessary that this HPC migrate to the intramedullary compartment of the bone marrow (BM) for the transplant to be effective, and that they be inserted and proliferate in the hematopoietic niche (HN). The HPC present in the biological material of the transplant are lodged and grow in the bone marrow (BM) to form blood cells, nevertheless, it is necessary that the HPC be evaluated for their immunophenotype and functional capacity before transplant.

The immunophenotype and the functional capacity are criteria which evaluate the quality of the transplanted material, such as: the total number of viable nucleated cells, the clonogenic capacity (colony forming units, CFU) and the human leucocyte antigen (HLA), which partially determine the time required for the hematopoietic progenitor cells (HPC) to grow in the bone marrow. The inter-individual variability in the quantity and function of the cells present in the biological material which is transplanted should be evaluated for each donation.

Thus, the viable cell content in the cellular populations presented in the bone marrow are established in the donor samples, peripheral blood (PB) and the umbilical cord blood (UCB) by the immune-phenotyping of the molecules CD34+ and CD45+ and the clonogenic trials which evaluate the potential of the cells to respond to growth factors and to proliferate to produce colony forming units (CFU). The correlation values of the cellular doses CD34+ and CFU per kilogram of weight of the patients in correspondence with these analyses are used to determine the results of the transplant (Castillo N, et al. 2015. Post-Thaw Viable CD45+ Cells and Clonogenic Efficiency are Associated with Better Engraftment and Outcomes after Single Cord Blood Transplantation in Adult Patients with Malignant Diseases. Biol Blood Marrow Transplant. Dec; 21 (12): 2167-2172).

The clonogenic analysis which indicates the proliferative capacity as a measure of the viability and the differentiation capacity of different hematopoietic lineages (functionality) of the cells, is typically determined by a method which involves the manual counting of the colony forming units under the microscope. That analysis generally varies with the observer and has high levels of error. Furthermore, obtaining the result takes, as a minimum, seven days for the cultivation of the cells. Even so the dosage of the CD34+ cells and of the CFU are currently the most precise measures available to predict the hematopoietic transplant capacity.

There have been multiple studies which evaluate the therapeutic potential of cells for application in cancer studies, cellular therapy, diagnosis, cellular migration, angiogenesis, embryogenesis, axion growth and immunology among others, as well as trial methods which determine the proliferative status of hematopoietic progenitor cells (HPC).

The United States Patent application US 2018/0236442 discloses trials which enable quantification of directed cellular migration, evaluation of the response to cellular migration and the response to the cellular adherence in cellular populations. This document also discloses a device to measure the response to cellular adhesion, which quantifies the directed adhesion response released by the presence of migration induction factors.

On the other hand, United States patent US 7,700,354 discloses test methods which determine the proliferative status of hematopoietic and progenitor of cells, as well as tests to evaluate compounds which modulate the growth of said cells and identification of subpopulations of the same, which are suited for transplant. In one embodiment, this patent discloses a trial to determine the proliferative status of a primitive hematopoietic cellular population as a function of the ATP content of the cells, wherein the detected level of ATP indicates the proliferative status of the primitive hematopoietic cells. In an additional embodiment, the method disclosed therein comprises putting the primitive hematopoietic cellular population in contact with at least one cytokine and optionally generating a cellular population which is substantially HPC enriched.

Additionally, the patent application US 2020/0268850 discloses methods and composition to mobilize HPC which comprises administering an effective quantity of an agent to a subject which inhibits the level or activity of Exostosin 1 (EXT1) in the subject and thus to mobilize the HPC and/or progenitor cells towards the peripheral blood of the subj ect.

Even if the state-of-the-art teaches alternative methods and devices to evaluate and promote cellular mobilization, there is still a necessity for methods which enable the evaluation of the proliferative activity and pre-transplant differentiation of the HPC with greater precision and in less time than the chromogenic trials which are currently in use. In addition, there is still a need for a technique which enables discerning the functionality of the low percentages of HPC in the sources used for transplant or of the neoplastic cells present in patients with leukemia.

The present invention represents a solution to said unmet need, since it provides a test which predicts the response capacity of the HPC to the specific stimuli of a hematopoietic niche (HN) which enables their nesting (also known as *"homing"*), which correlates with their colony forming potential. Furthermore, in the case of specimen of patients with leukemia, the response capacity to the hematopoietic niche (HN) stimuli could contribute to predicting the invasive capacity of the neo-plastic cells to said NH. Additionally, the method which was developed could be employed as a purification method of the cells which specifically migrate towards the NH, for clinical applications.

### SUMMARY OF THE INVENTION

The present invention relates to an *in vitro* method to quantify and evaluate the activity of a source of viable hemopoietic progenitor cells (HPC), which comprises the steps of:
a) incubating an adhesion solution on a surface or adhesion compartment; b) add a migration buffer to a chemotactic compartment which comprises a chemotactic agent; c) suspend the cellular phase of a source of HPC in a migration buffer and incubation thereof in a cellular compartment; d) recover the content of the chemotactic compartment and count the cells which migrated; wherein the cellular compartment is separated from the chemotactic compartment by an adhesion surface or adhesion compartment; and wherein the cellular compartment and the chemotactic compartment are in mutual communication via a continuous solution which comprises a chemotactic gradient.

### BRIEF DESCRIPTION OF THE DRAWINGS

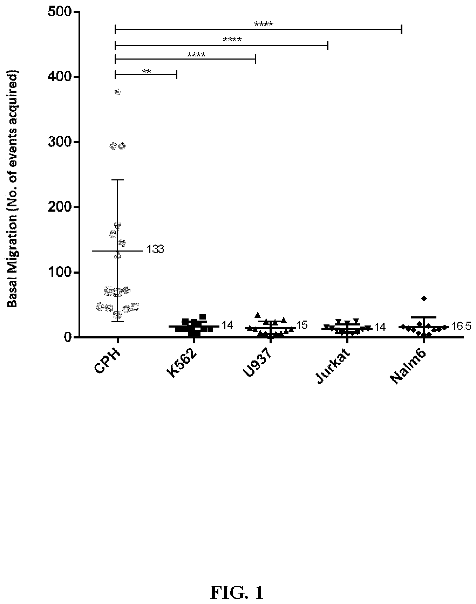

**FIG. 1** Chemokinesis of hematopoietic progenitor cells (HBC) from donors of umbilical cord blood (UCB) and strains of leukemia. The cells are seeded into Trandswell trays and are incubated for three hours to determine their migration without stimulus (RPMI 1640, BSA 2%). This demonstrates the number of events resulting from cytometric analysis of the flux of the HPC (n=15) of 12 donors of UCB and of the K562 (n=11), U937 (n=13), Jurkat (n=12) and Nalm6 (n=12) cell lines. Each HPC is represented by a geometric figure. Replication was performed on three HPC donors. The statistical analysis was performed with the Kruskal-Wallis test for nonparametric methods: p <_ 0,01(**) y p ≤ 0,0001(****). The data was analyzed in the GraphPad Prism 6 program.

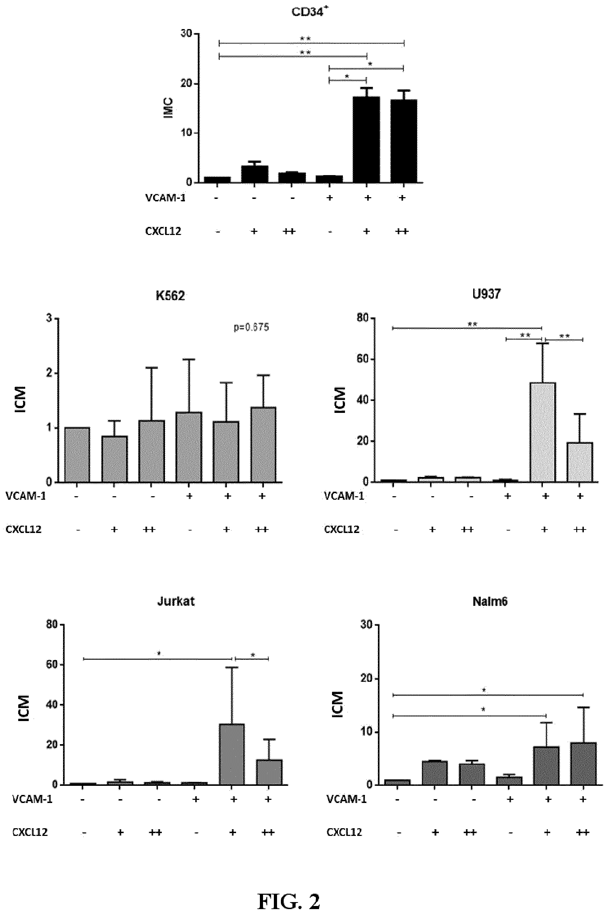

**FIG. 2** Chemotaxis and haptotaxis induced by the individual stimuli and combination of VCAM-1 and CXCL12 in HPC of donors of UCB and leukemia strains. The ICM of the HPC (n=8 donors) and that of the leukemia strains (K562, U937, Nalm6 and Jurkat) were compared with the same number of experiments (n=4) to that of two different concentrations of CXCL12 (50 ng/mL [+] and 100 ng/mL [++]) and 10 µg/mL of VCAM-1 [+]. The Friedman statistical analysis test: p <_ 0.05 (*) y p ≤ 0.01(**) indicates statistical significance. The data was analyzed in the GraphPad Prism 6 program.

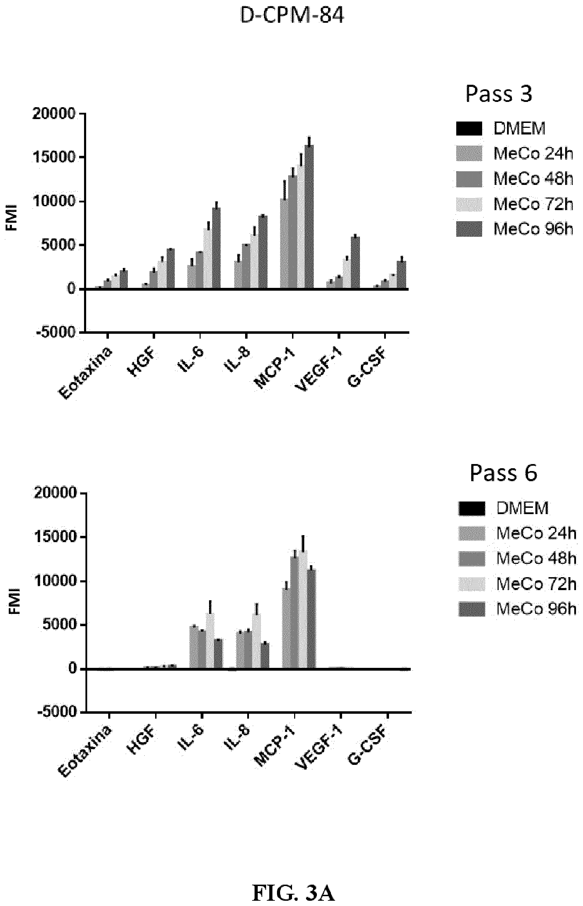

**FIG. 3A** Characterization of the chemokines of mesenchymal stromal cell conditioned media (MeCoCEM). Comparison of the fluorescent media intensities (FMI) of chemokines and growth factors secreted in the MeCoCEM of donor 1 (D-CPM-84) after 24, 48, 72 and 96 hours of cultivation in the passes 3 (left) and 6 (right).

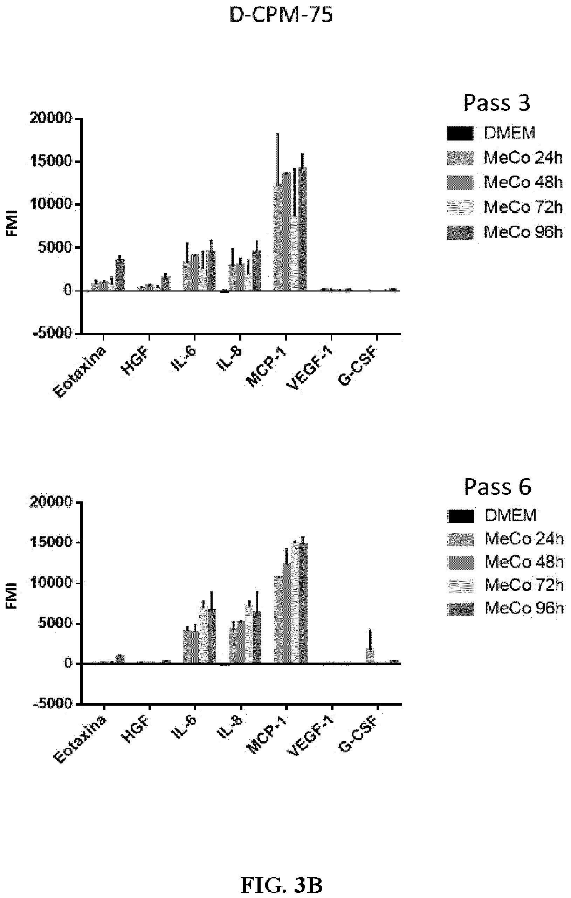

**FIG. 3B** Comparison of the fluorescent media intensities (FMI) of chemokines and growth factors secreted in the MeCoCEM of donor 2 (D-CPM-75) after 24, 48, 72 and 96 hours of cultivation in the passages 3 (left) and 6 (right).

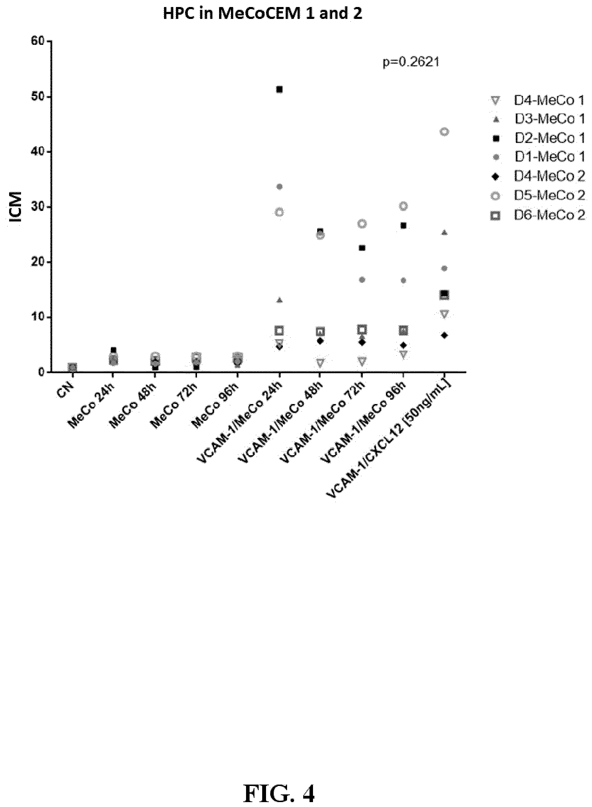

**FIG. 4** Comparison of the ICM of donors to MeCoCEM and VCAM-1/ MeCoCEM. The ICM of the HPC of distinct UBC donors in response to MeCoCEM collected after 24, 48, 72 and 96 hours of cultivation, including the combinations of MeCoCEM with VCAM-1 (10 µg/mL) and VCAM-1 (10 µg/mL) with CXCL12 [50 ng/mL]. The data was analyzed in the GraphPad Prism 6 program.

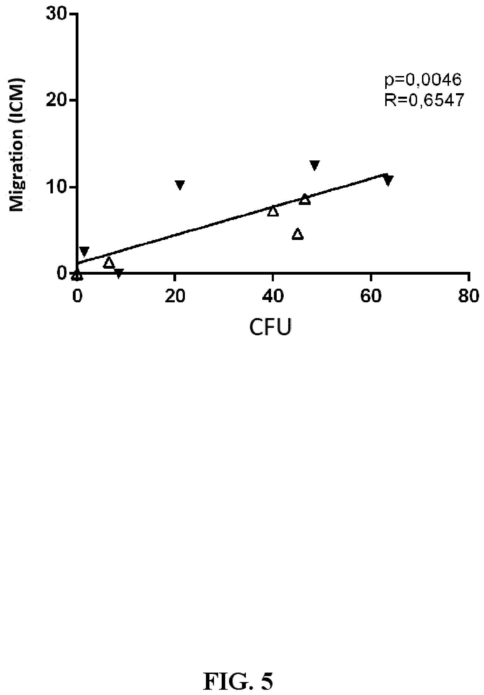

**FIG. 5** The correlation between clonogenicity and ICM of mononuclear cells (MNC) of UCB. The colony forming units (CFU) where the averages of the duplicated reading of the wells of two distinct donors of MNC from UCB. The ICM was evaluated in the MNC of different donors of UCB. Combinations of the functional biomarkers VCAM 1 (10 µg/mL), CXCL12 (100 ng/mL) and the MeCoCEM of UCB of different donors were employed. The statistical analysis was performed by means of a linear regression with a p = 0.0046 and the correlation coefficient of R= 0.6547. The graphs represent Donor 1 (black triangle) and Donor 2 (white triangle). The data was analyzed in the GraphPad Prism 6 program.

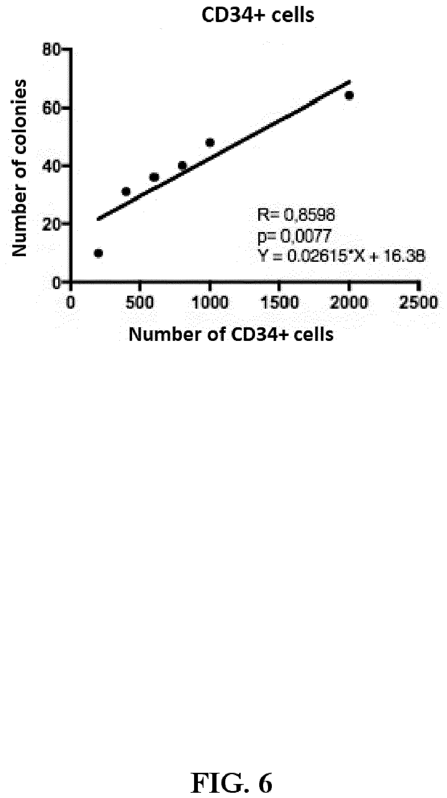

**FIG. 6** Curve of the titration of clonogenicity according to the number of CD34⁺ cells seeded. They were seeded to each well with the medium MethoCult (STEMCELL^{™} TECHNOLOGIES) without EPO and Iscove Modified Dulbecco Media (IMDM) supplemented with 2% of bovine fetal serum (BFS) with between 200 and 1000 CD34⁺ cells/well as determined by the Stem Cell Enumeration (Becton Dickinson) by donor. Cells of different donors were seeded in a cultivation tray of six wells and were cultivated for 14 days at 37°C with an atmosphere of 95% humidity and 5% of CO2. The counting of CFU was performed by two independent observers.

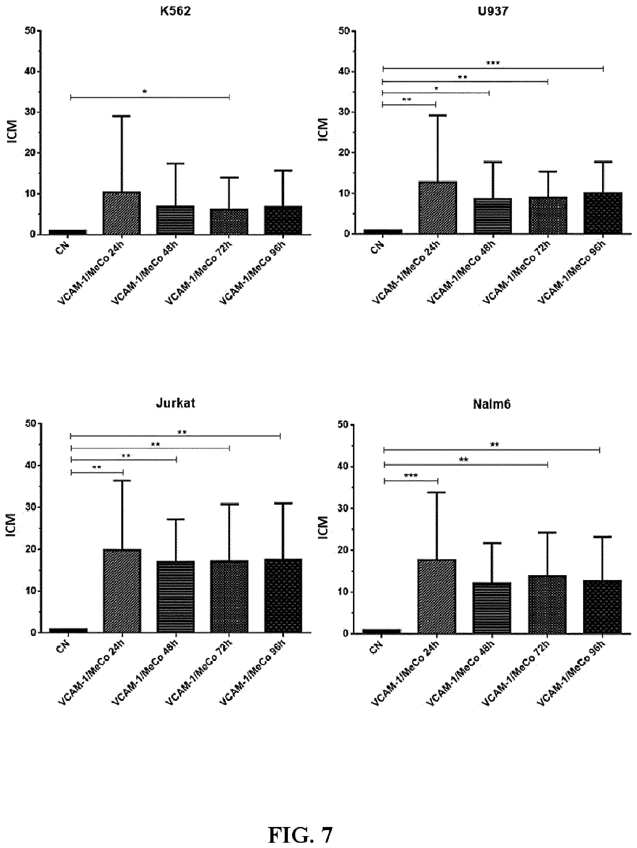

**FIG.** 7 The ICM of leukemia strains (n=7) compared with the VCAM-1 with MeCoCEM of donors 1 and 2, collected after every 24 hours of cultivation. The Friedman statistical analysis test: p <_ 0.05 (*), p ≤ 0.01(**) and p < 0.001(***) indicates statistical significance. The data was analyzed in the GraphPad Prism 6 program. The standard error of the mean (SEM) is represented in the graphics.

### DETAILED DESCRIPTION OF THE INVENTION

For the purposes of interpreting the terms used throughout the present document, their usual meaning in technical fields should be borne in mind, unless a particular definition is incorporated or the context clearly indicates to the contrary. Additionally, the terms utilized in their singular form also include the plural form.

The present invention relates to an *in vitro* method for the quantification and evaluation of the activity of a viable source of hematopoietic progenitor cells (HPC), which comprises incubating an adhesion solution on a surface or in an adhesion compartment; adding a migration buffer which comprises a chemotactic agent to the chemotactic compartment; suspending the cellular phase of a source of HPC in a migration buffer and incubating the source of HPC in the cellular compartment; recovering the content of the chemotactic compartment and counting the cells which migrated; wherein the cellular compartment is separated from the chemotactic compartment via the adhesion surface or adhesion compartment; and wherein the cellular compartment and the chemotactic compartment are in mutual communication via a continuous solution which comprises a chemotactic gradient.

Bearing in mind the confluence of certain intracellular signaling routes in self-renewal, proliferation and cellular migration of HPC, it can be inferred which stimuli of the HN induce the function and hematopoietic localization thereof in a simultaneous manner. Moreover, the invasive and proliferative capacity of leukemia cells could respond to this same pattern. Evidence in the primate model in which there is molecular blockage of adhesion by the vascular cell adhesion-1 molecule (VCAM-1) or the intercellular adhesion molecule-1 (ICAM-1) and chemotaxis, like chemokine 12 with C-X-C (CXCL12) induce a mobilization of the HPC from the bone marrow to peripheral blood, (Sahin AO, Buitenhuis M. 2012. Molecular mechanisms underlying adhesion and migration of hematopoietic stem cells. Cell Adh Migr. Jan-Feb;6(1):39-48. doi: 10.4161/cam. 18975. PMID: 22647939; PMCID: PMC33641362), would enable the conclusion that a functional evaluation of these cells in response to stimuli could enable the detection, classification and isolation thereof by their therapeutic potential.

The method of the present invention enables evaluation of the content of cells of a HPC source which have colony forming potential, and which at the same time is related to the migratory capacity of said cells to certain stimuli of the HN. Moreover, the method enables the selection and quantification of the HPC in a manner equivalent to that in which detection is performed in the clonogenicity tests. The method developed here could be converted to a functional pretransplant quantification method of HPC, which could be automated and be precise for the evaluation of different sources of HPC. In fact, the migratory capacity of HPC could be employed for the purposes of separating HPC from mixtures of cells and the recovery thereof for use in cellular therapy, in such a manner that the method described herein could be employed to purify HPC from different sources for the clinical use thereof.

Moreover, bearing in mind that the migratory capacity of leukemia cells or tumors is related to the malignancy of these pathologies, the method developed here could also be used as a prognostic functional biomarker that permits determination of the capacity of the leukemia cells or tumors to invade the HN and displace the HPC.

For the purposes of the present invention, a *"source of hemopoietic progenitor cells"* refers to sources for obtaining HPC, among which are bone marrow (BM), mobilized peripheral blood (MPB) or umbilical cord blood (UCB). In one embodiment of the present invention, the source of HPC could be mononuclear cells isolated from any of the above sources. In a particular embodiment, the source of the HPC is the UCB.

The HPC could be fresh pre-transplant cells or defrosted pre-transplant cells. In accordance with the method of the present invention, the HPC could be isolated by any known method which concentrates the HPC, for example, isolation techniques such as: use of automated equipment (Sepax^{®}, AXN^{®}), isolation of mononuclear cells (MNC) by density gradients, isolation with immunomagnetic beads or cellular separation by flow cytometry (sorting); these latter two by positive selection using antibodies to specific molecules of HPC such as: CD34⁺, CD133⁺ or by negative selection by antibodies of a specific lineage.

In one particular embodiment of the present invention the isolates of MNC and HPC (immunomagnetic isolation of CD34⁺, Milteyi) is confirmed using absolute counting of CD34⁺/CD45⁺ cells using the Stem Cell Enumeration kit in TruCount tubes (Beckton Dickinson) by the clinical application FACSCanto Software (BD, Becton Dickinson) of the FACSCantoII cytometer (Becton Dickinson).

In accordance with the present invention, an *"adhesion solution"* should be understood to be a solution which contains cellular adhesion molecules, that is to say, those which mediate the cell-to-cell adhesion or the adhesion of cells with the extracellular matrix. As is known by any person moderately versed in the subject, the adhesion molecules comprise four big families, that is to say, the family of integrins, of immunoglobulins, of selectins and cadherins.

In one embodiment of the present invention, the adhesion solution comprises molecules selected from among vascular cell adhesion-1 (Vascular cell adhesion molecule-1, VCAM-1), intercellular adhesion molecule-1 (Intercellular AdhesionMolecule-1, ICAM-1) laminin, fibronectin, collagen and mixtures of the same. In one particular embodiment, the adhesion solution comprises VCAM-1.

Said adhesion molecules can be at a concentration of the order of pg/mL to mg/mL in the adhesion solution. In one embodiment of the invention, the concentration of the adhesion molecules is of the order of µg/mL. In a particular embodiment, the concentration of said molecules is between 1 and 100 µg/mL, or between 1 and 50 µg/mL or between 50 and 100 µg/mL. In another particular embodiment, the concentration is between 5 and 20 µg/mL. In an even more particular embodiment, the concentration of the adhesion molecules is 10 µg/mL.

In one embodiment of the invention, the adhesion solution additionally comprises phosphate buffered-saline (PBS). In a particular embodiment, the concentration of PBS is 1X.

For the purpose of the present invention, the *"migration buffer"* is a composition in which the cells are suspended to complete the migration process. In one embodiment of the present invention, the migration buffer comprises the Roswell Park Memorial Institute medium (RPMI) and bovine serum albumin (BSA) or human serum albumin (HSA). In a particular embodiment, the migration buffer comprises a chemotactic agent. A "chemotactic agent" corresponds to a substance which induces the HPC to migrate towards the HN. Thus, the *chemotactic agent* is selected from among, without limitation to the chemokine denominated CXCL12 (a factor derived from stromal cells, *Stromal cell-derived factor-1,* o SDF-1), *Stem Cell Factor* (SCF), *Hepatocyte growth factor* (HGF), *Vascular Endothelial Growth Factor* (VEGF), interleukin 8 (IL-8), interleukin 6 (IL-6), *FMS-like tyrosine kinases 3 ligand,* FLT 3L), *chemokine (C-C motif) ligand 2* (CCL-2), thrombopoietin (TPO), *Granulocyte colony-stimulating factor* (G-CSF), *Granulocyte-macrophage colony-stimulating factor* (GM-CSF) and mesenchymal stomal cell conditioned media (MeCoCEM), or mixtures thereof.

For the purpose of the present invention, the *"mesenchymal stromal cell conditioned media"* (MeCoCEM) refers to media obtained from *in vitro* cultures of mesenchymal stromal cells (MSC) which have secreted soluble factors and signaling molecules to said media which are typically used for growth. In a particular embodiment of the present invention the migration buffer comprises MeCoCEM of Wharton gelatin of umbilical cord which were cultured in Dulbecco's Modified Eagle's Medium (DMEM) without serum for 24, 48, 72 and 96 hours. In another particular embodiment, the MeCoCEM contain factors such as, but not limited to, IL-6, IL-8, MCP-1, HGF, VEFG, G-CSF, among others.

In accordance with the present invention, the cellular compartment, the adhesion compartment and the chemotactic compartment correspond to locations/sections of whatever known device in the prior art which permits cellular migration, for example, selected from among, without particular limitation, a *Transwell* tray, migration chambers based on microfluids, Boyden chambers, Dunn chambers or capillary techniques. In any event, in the device, the cellular compartment and the chemotactic compartment are separated by means of the adhesion compartment or adhesion surface, which enables fixing the adherent substrate (Haptotactic) and enables passage of the cells there through. On that occasion, the compartments of the device mutually communicate via a continuous solution which consists of the migration buffer which comprises a chemotactic gradient.

For the purposes of the present invention, a *"chemotactic gradient"* corresponds to a concentration gradient of the chemotactic stimulus which is generated in the migration buffer due to the presence of the chemotactic stimulus in the migration buffer of the chemotactic compartment, and the lack thereof in the migration buffer of the cellular compartment. Thus, the migration of the cells is determined by said concentration gradient of the chemotactic stimulus, from a lesser concentration of the chemotactic stimulus in the migration buffer in which the cells are found suspended (cellular compartment), towards a greater concentration of said stimulus in the migration buffer of the chemotactic compartment.

In a particular embodiment, the cellular compartment and the chemotactic compartment correspond to the upper and lower chambers of a *Transwell* tray which are in communication via a porous membrane, with the pore size of between 5 and 8 µm, which constitutes the adhesion compartment. The size of the membrane pores should enable migration therethrough of the HPCs deposited thereon.

Thus, the incubation stage of the adhesion solution consists in preparing the adhesion compartment by adding the adhesion solution and incubating for 12 to 24 hours at 37°C with an atmosphere of between 3 and 5% CO2, so that the adhesion molecule is fixed on the surface of the adhesion compartment.

Additionally, the incubation of the cellular phase of the source of the HPC in the migration buffer in this cellular compartment occurs for 2 to 5 hours at 37°C with an atmosphere of between 3 and 5% CO2. More particularly, the incubation conditions of this stage are an incubation for three hours at 37°C with 5% CO2.

Finally, in accordance with the method of the present invention, the cells which migrate from the cellular compartment towards the chemotactic compartment and which are recovered from the chemotactic compartment can be counted by any of the known methods in the prior art, such as fluid cytometry, hematocytometry, or counting in a Neubaur chamber.

In a particular embodiment of the present invention, the method comprises: incubating an adhesion solution in the upper chamber of a Transwell tray; the addition of migration buffer which comprises a chemotactic agent to the lower chamber; dispense HPC suspended in migration buffer in the upper chamber and incubate; collect the content of the lower chamber and distinguish the cells of the same donor which migrated with or without stimulus. In a particular embodiment, a volume of the upper chamber is transferred to cytometric tube to perform the count of the absolute number of the cells which migrated. The quantity of events (# events) is registered by cytometry of the flux.

In an additional embodiment of the present invention, the method enables evaluation of cellular strains of human cancers and extrapolation of the results to cellular therapy. In a particular embodiment, the cellular strains include, without honey particular limitation, K562, U937, Jurkat and Nalm6.

The present invention will be expanded in detail by means of the following examples, which are provided solely for illustrative purposes and not with the object of limiting the invention.

### EXAMPLES

### Example 1. Molecular characterization of conditioning media of CEM

The CEM isolated from gelatin of Wharton of the umbilical cord of different donors are cultivated from the zero pass in supplemented (10% of inactivated Bovine Fetal Serum (BFS) (Gibco ^{®}) *Dulbecco's Modified Eagle's medium* (DMEM) (Gibco ^{®}) 100 µg/mL of streptomycin and 100 U/mL of penicillin) until reaching a confluence of 80 to 90% in different passes (3 to 6). The cultures were washed with dilute sterile PBS 1X, pH 7.4 (Gibco ^{®}) to eliminate residues of the supplemented DMEM. The cells were maintained in culture in DMEM 1X medium (without supplementation) and there were incubated for 96 hours at 37°C, with an atmosphere of 5% CO2. The cultures were observed by an inverted microscope (Leica Microsystems, USA) every 24 hours and the MeCoCEM were collected in conical 50 mL tubes (Corning^{®}) in different cellular passes. The MeCoCEM were centrifuged at 3000 g for 10 minutes in a refrigerated centrifuge, filtered (pores of 0.45 µm) to reduce the presence of detritus and were stored at -80°C. The MeCoCEM were characterized with the *Human Cytokine 30-Plex panel kit* (Invitrogen^{™}) using duplicates of every condition. The analysis of the samples was performed using the Luminex^{®}200^{™} System, according to the makers instructions, and the analysis of the data was performed using *Graph Pad Prism* 6 program (San Diego, CA, USA), averaging the duplicated values of the intensities of the fluorescent media (FMI) detected by the team in each MeCoCEM. Seven factors were detected (Eotaxina, HGF, IL-6, IL8, MCP-1, VEGF-1 and G-CSF) of the 30 analyzed with Luminex from the reading of the kit. A discrete increase of the signal of these factors was observed as the duration of culture advanced for the recovery of the MeCo in CEM from the third to the sixth pass (Fig. 3).

### Example 2. Basal migration or chemokinesis of donors of HPC and of the leukemia strains

For the basal migration tests or the chemokinesis, a migration buffer composed of RPM I-1640 with additional 2% BSA (without chemotactic stimulus) were incubated in the lower chamber to activate the *Transwell* membrane (pores of 5 µm, Corning Inc., Costar^{®}, USA) which were preincubated without adhered stimulus. After the incubation, 50,000 cells of CD34+, K562, Nalm6, Jurkat and U937 were seeded, with each cellular line suspended in migration buffer in its respective upper chamber. After three hours of incubation at 37°C an atmosphere of 5% CO2, the absolute number of the cells which migrated to the lower chamber were counted. For this purpose, a volume of 500 µL of samples were transferred from the lower chamber to us cytometric tube. The number of events (#events) was acquired by flow cytometry under the following conditions: threshold: 25,000, flow rate: high, stopping time: 60 seconds, events record: 100,000 and voltages: FSC: 260/SSC: 375. (FACSCantoII Becton Dickinson) via the FACSDiva Software program (BD, Becton Dickinson).

This CD34+ cells were isolated from 12 donors with the CD34+ microbeads human kit (Miltenyi Biotec) according to the makers instructions from the mononuclear cells isolated by a density gradient. The purity of the isolates was confirmed to be HPC with more than 90% of the CD34 cells marked with the antibody anti-CD34+ PerCP-Cy 5.5 (Clon 8G12, BD, Class II epitope). The cellular data were acquired and analyzed as indicated above. It was observed that the number of events acquired as basal migration or chemokinesis was greater in CD34+ cells isolated from the distinct donors of UCB than in the leukemia cell lines K562, Jurkat, Nalm6 and U937. A variation of the migration depending on the donor was observed which was confirmed in the duplicates which were performed with CD34+ cells isolated from the same donor which exhibited a similar number of events (see figure 1, rhombus, square and empty circle). The value of the number of events acquired by fluid cytometry in chemokinesis tests (migration without stimulus) was used as a common denominator of the index of cellular migration (ICM), which indicates the number of times that basal migration was induced by haptotactic and chemiotactic stimuli, according to the formula ICM = Number of counted cellular events induced by haptotaxis or chemotaxis, or both stimuli/number of events of chemokinesis counted (without stimulus).

### Example 3. Indices of Cellular migration after stimulation by VCAM-1 and CLCX12 in HPC donors and in leukemia strains

These were performed in accordance with the procedure described in example 2. For these migration trials, the upper chamber of the well of the tray was incubated with 50 µL of the adhesion solution composed of the recombinant protein VCAM-1 (Sigma-Aldrich, Life Science, USA) at a concentration of 10 µg/mL overnight at 37°C with an atmosphere of 5% CO2.

After the incubation, 50,000 cells of CD34+, K562, Nalm6, Jurkat and U937 were seeded with each cellular line suspended in migration buffer in its respective well.

600 µL of the RPMI medium (migration buffer) with recombinant CXCL12 (SDF-1) (R&D systems(R), Minneapolis, USA) were added at concentrations of 50 and 100 ng/mL.

Migration buffer without chemotactic agent was seeded to the lower chamber as a chemotaxis negative control. The cells were incubated for three hours at 37°C with an atmosphere of 5% CO2.

After three hours at 37°C in an atmosphere of 5% CO2, the absolute number of the cells which migrated to the lower chamber were counted.

The same procedure as in example 2 was implemented to determine the count of the absolute number of cells which migrated. The ICM was calculated with the number of counted cellular events induced by haplotaxis or chemiotaxis, or both stimuli/number of counted events of chemokinesis (without stimulus) - obtained according to example 2. The combination of the haptotactic stimulus of VCAM-1 and the chemotactic stimulus of CLCX12 induces an ICM of the HPC (CD4 cells) of 20 times [greater] on average for the different donors. The cellular strains U937 and Jurkat exhibited indices of up to 60 times the basal migration, which tended to reduce to 20 times when greater concentrations of CXCL12 (100 ng/mL, (++)) were used. The cell strain Nalm6 Exhibited a behavior similar to that of HPC, while the cell line K562 did not respond to the same stimuli. The cellular strain Nalm6 could serve as a positive control and that of K562 as a negative control of the functional test of HPC (Fig. 2).

### Example 4. Indices of celular migration of HPC resulting from VCAM-1 stimuli and CEM conditioned media

The trial was conducted just as described in examples 2 and 3. The adhesion solution composed of the recombinant protein VCAM-1 was incubated at a concentration of 10 mcg/mL overnight at 37°C with the 5% CO2 atmosphere. The controls without adhesive stimulus were incubated with the migration buffer composed of RPMI-1640 in addition to 2% BSA (migration buffer) and in the case of the positive control, in a well without a membrane (hypothetic positive control = 100% migration). The CD34+ cells of different donors were suspended in migration buffer in the upper chamber, either preincubated or not, with adherence stimulus. Some 50,000 CD34+ cells were seeded per trial for every donor of HPC, and represented by a geometric figure in Fig. 4. The MeCoCEM were added to the lower chamber which is the cultivation media DMEM without bovine fetal serum (BFS), and conditioned over 24, 48, 72 and 96 hours with CEM in accordance with example 1, The MeCoCEM was isolated from the CEM in the third pass of the cellular culture. The use of MeCoCEM as combined chemotactic agents with the haptotactic stimulus of VCAM-1 differentially induced the HPC migration in a similar manner to that induced by the combination of the haptotactic VCAM-1 and the chemotactic CXCL12. The interindividual variation of the ICM enabled the discernment between the migratory capacity of the HPC of the distinct donors while the consistency of the interindividual ICM enabled the establishment of the definition of HPC donors of high or low migrator re-potential. The MeCoCEM were collected from the donors (MeCoCEM 1 and MeCoCEM 2) of CEM cultivated in the third pass (Fig. 4).

### Example 5. Correlation between trials of clonogenicity and MNC from UCB migration trials

MNC Isolated by density gradient of Ficoll-Hypaque which were subject to trials of migration were collected from the lower chambers after three hours of the incubation described in examples 2, 3 and 4. A 500 µL sample was collected from the total volume of the migration buffer of the lower chamber of Transwell to implement the calculation of the ICM and 20 µL was seeded in the MethoCult without EPO medium (STEMCELL^{™} TECHNOLOGIES) in plaques of 12 wells per culture (Corning Inc. Costar^{®}, USA) and were cultivated over 14 days at 37°C with the 95% humidified atmosphere and 5% of CO2. The count of the colony forming units was conducted by two independent observers. A correlation carved between ICM and UFC was prepared per donor. One donor was represented by a filled triangle and the other by an empty triangle (Fig. 5).

### Example 6. Clonogenicity titration curve of HPC

Some 200, 400, 600, 8000 and 1,000 CD34+ cells per well were inoculated per donor into MethoCult (STEMCELL^{™}TECHNOLOGIES) without EPO but with IMDM supplemented with 2% of bovine fetal serum (BFS), according to the absolute count of CD34⁺ cells determined by the Stem Cell Enumeration kit (Becton Dickinson). Cells of different donors were seeded into a culture tray of six wells and cultivated for 14 days at 37°C with a 95% humidified atmosphere and 5% CO2. The UFC count was implemented by two independent observers. The correspondence between the number of CD34⁺ cells and the capacity to form colonies were observed, which permitted the calculation of the quantity of CD34⁺ cells which correspond to the ICM obtained according to example 5 (Fig. 6).

### Example 7. Indices of celular migration of leukemia cells on stimulation by VCAM-1 and CEM conditioned media.

The same experimental design as employed in example 4 was used and it was observed that the Jurkat, U937 and Nalm6 cells responded in a significant manner to the induction by MeCoCEM collected at the distinct durations of culture, as can be observed in Fig. 7. This leads to the conclusion that the method of the present invention could contribute to detect the invasive capacity of the neoplastic cells to HN.

## Claims

1. An *in vitro* method to quantify and evaluate the activity of a source of viable hemopoietic progenitor cells (HPC), which comprises the steps of:
a) incubating an adhesion solution on a surface or adhesion compartment;
b) add a migration buffer to a chemotactic compartment which comprises a chemotactic agent;
c) suspend the cellular phase of a source of HPC in a migration buffer and incubation thereof in a cellular compartment;
d) recover the content of the chemotactic compartment and count the cells which migrated;
wherein the cellular compartment is separated from the chemotactic compartment by an adhesion surface or adhesion compartment; and
wherein the cellular compartment and the chemotactic compartment are in mutual communication via a continuous solution which comprises a chemotactic gradient.

2. The method in accordance with claim 1, wherein the adherence solution comprises adhesion molecules selected from among the vascular cell adhesion molecule-1 (VCAM-1), the intracellular adhesion molecule-1 (ICAM-1), laminin, fibronectin, collagen and mixtures thereof.

3. The method in accordance with claim 2, wherein the concentration of the adhesion molecules is of the order of pg/mL to Mg/mL.

4. The method in accordance with claim 2, wherein the adhesion solution additionally comprises phosphate buffered saline (PBS) 1X.

5. The method in accordance with claim 1, wherein the migration buffer comprises Roswell Park Memorial Institute (RPMI) medium and bovine serum albumin (BSA) or human serum albumin (HSA).

6. The method in accordance with claim 5, wherein the concentration of the BSA or HAS at a concentration of between 1 and 3% in the RPMI media.

7. The method in accordance with claim 1 wherein the chemotactic agent is selected from among the factor derived from stromal cells-1, (CXCL12 or SDF-1), stem cell factor (SCF), hepatocyte growth factor (HGF), vascular endothelial growth factor (VEGF), interleukin 8 (IL-8), interleukin 6 (IL-6), FMS-like tyrosine kinase 3 ligand (FLT3L), the chemotactic agent is selected from among), chemokine ligand 2 (CCL-2), thrombopoietin (TPO), granulocyte colony-stimulating factor (G-CSF), granulocyte-macrophage colony-stimulating factor (GM-CSF) and mesenchymal stomal cell conditioned media.

8. The method in accordance with claim 1 wherein the HPC are incubated for 2 - 5 hours at 37°C with an atmosphere of between 3 and 5% of CO2.
